# EUROPEAN PATENT SPECIFICATION

(11) **EP 3 212 228 B1**
(45) Date of publication and mention of the grant of the patent: **03.12.2025**
(21) Application number: 15794838.1
(22) Date of filing: 27.10.2015
(51) Int. Cl.: A61K 31/46, A61K 31/337, A61K 31/513, A61K 31/519, A61K 31/555, A61K 31/7068, A61K 38/17, A61K 39/395, A61K 45/06, A61P 1/04, A61P 1/16, A61P 1/18, A61P 11/00, A61P 13/12, A61P 15/00, A61P 35/00, A61P 35/04, A61P 43/00

(54) **MARAVIROC ALONE OR IN COMBINATION THERAPY FOR THE TREATMENT OF CANCER**
MARAVIROC ALLEIN ODER IN KOMBINATIONSTHERAPIE ZUR BEHANDLUNG VON KREBS
MARAVIROC SEUL OU EN THÉRAPIE COMBINÉE POUR LE TRAITEMENT DU CANCER

(30) Priority: 27.10.2014 US 201462068919 P
(43) Date of publication of application: 06.09.2017
(73) Proprietor: Ruprecht-Karls-Universität Heidelberg, 69117 Heidelberg (DE)
(72) Inventor: HALAMA, Niels, 94342 Seeheim-Jugenheim (DE); JÄGER, Dirk, 69121 Heidelberg (DE); ZOERNIG, Inka, 69120 Heidelberg (DE); GRABE, Niels, 69120 Heidelberg (DE)
(74) Representative: Zwicker, Jörk
(86) International application number: PCT/EP2015/074850
(87) International publication number: WO 2016/066634

(56) References cited:
- US-A1- 2013 303 512
- MENCARELLI ANDREA ET AL: "CCR5 Antagonism by Maraviroc Reduces the Potential for Gastric Cancer Cell Dissemination", TRANSLATIONAL ONCOLOGY, vol. 6, no. 6, 1 December 2013 (2013-12-01), United States, pages 784 - 793, XP093127433, ISSN: 1936-5233, DOI: 10.1593/tlo.13499
- LAURA OCHOA-CALLEJERO ET AL: "Maraviroc, a CCR5 Antagonist, Prevents Development of Hepatocellular Carcinoma in a Mouse Model", PLOS ONE, vol. 8, no. 1, 9 January 2013 (2013-01-09), pages e53992, XP055385231, DOI: 10.1371/journal.pone.0053992
- KAST R E: "Glioblastoma: Synergy of growth promotion between CCL5 and NK-1R can be thwarted by blocking CCL5 with miraviroc, an FDA approved anti-HIV drug and blocking NK-1R with aprepitant, an FDA approved anti-nausea drug", JOURNAL OF CLINICAL PHARMACY AND THERAPEUTICS, vol. 35, no. 6, December 2010 (2010-12-01), BLACKWELL PUBLISHING LTD GBR, pages 657 - 663, XP002752367, ISSN: 0269-4727
- ANONYMOUS: "Prepped By Tumor Cells, Lymphatic Cells Encourage Breast Cancer Cells to Spread", 3 September 2014 (2014-09-03), XP002752368, Retrieved from the Internet <URL:http://www.hopkinsmedicine.org/news/media/releases/prepped_by_tumor_cells_lymphatic_cells_encourage_breast_cancer_cells_to_spread> [retrieved on 20151217]
- VELASCO-VELÁZQUEZ MARCO ET AL: "CCR5 antagonist blocks metastasis of basal breast cancer cells.", CANCER RESEARCH, vol. 72, no. 15, 1 August 2012 (2012-08-01), pages 3839 - 3850, XP002752332, ISSN: 1538-7445
- ANONYMOUS: "CCR5-blockade in Metastatic Colorectal Cancer (MARACON)", 1 October 2014 (2014-10-01), XP002752333, Retrieved from the Internet <URL:https://clinicaltrials.gov/ct2/show/NCT01736813?term=NCT01736813&rank=1> [retrieved on 20141217]
- CAMBIEN BÉATRICE ET AL: "CCL5 neutralization restricts cancer growth and potentiates the targeting of PDGFR[beta] in colorectal carcinoma.", PLOS ONE, vol. 6, no. 12, E28842, 2011, pages 1 - 11, XP002752334, ISSN: 1932-6203

## Description

The present invention relates to CCR5 antagonists for use in the treatment of metastatic colorectal cancer in a subject receiving immunomodulatory therapy with anti-PD-1. The invention also relates to CCR5 antagonists for use in the treatment of metastases of colorectal carcinoma by inducing tumor cell death.

### BACKGROUND OF THE INVENTION

Cancer cells are cells that are transformed and as such are characterized by uncontrolled and excessive growth as well as in many instances by invasive behavior, destroying healthy tissues. In the course of the diseases cancer cells do interact with the host's immune system and the host's immune cells as well as all other cells present in the microenvironment of an organ. Immune cells have an important role as they are able to destroy cancer cells and therefore the mechanisms by which the tumor protects itself from the immune system are manifold. An immune response requires the coordinated interaction of different subtypes of immune cells, ultimately leading to the destruction of cancer cells. The signaling or coordinating molecules involved are cytokines and chemokines. These contribute to an anti-tumoral or a pro-tumoral situation within the local microenvironment. Evasion mechanisms by cancer cells involve the tweaking of the cytokine signals to avoid destruction by the immune system. Standard therapies like chemotherapy and radiation not only rely on the direct killing of cancer cells but also on the immunological effects in the wake of such a therapy. Changing the local microenvironment from a pro-tumor situation into an anti-tumoral (immune system supporting) microenvironment is therefore a very promising approach to (a) kill tumor cells directly by withdrawing cytokines related to cancer cell growth (in metastases, in the primary tumor or in disseminated cancer cells) or (b) indirectly by leading to an anti-tumoral microenvironment that is receptive for effective immune responses induced by chemotherapy, radiation or other therapies. This situation is even more urgent for patients that had anti-tumoral treatment and did not have benefits from these prior treatments (therapy failure). In such a setting novel therapies or combinations are desperately needed to treat patients, prolong survival or induce a reduction of the tumor burden.

NCT01736813 (https:// clinicaltrials.gov/study/NCT01736813?term=NCT01736813&rank=1&tab=history&a=4#version-content-panel) titled "Treatment of Advanced Colorectal Cancer Patients With Hepatic Liver Metastases Using the CCR5-Antagonist Maraviroc (Phase I Maracon)" announces a clinical trial investigating the treatment of advanced colorectal cancer patients with Maraviroc.

There is a need in the art for improved means and methods for treating cancer, such as for improved means and methods for inducing a response to existing or novel anti-cancer therapies.

The inventors surprisingly found that CCR5 antagonist Maraviroc, which has previously been used to treat HIV infection, can be used in subjects receiving immunomodulatory therapy with anti-PD-1 to treat metastatic colorectal cancer.

### SUMMARY OF THE INVENTION

In a first aspect the present invention relates to a CCR5 antagonist for use in the treatment of metastatic colorectal cancer in a subject receiving immunomodulatory therapy with anti-PD-1, wherein said CCR5 antagonist is Maraviroc or [3H]maraviroc.

In a second aspect the present invention relates to a CCR5 antagonist for use in the treatment of metastases of colorectal carcinoma by inducing tumor cell death, wherein said CCR5 antagonist is Maraviroc or [3H]maraviroc.

This summary of the invention does not necessarily describe all features of the present invention. Other embodiments will become apparent from a review of the ensuing detailed description.

### BRIEF DESCRIPTION OF THE DRAWINGS

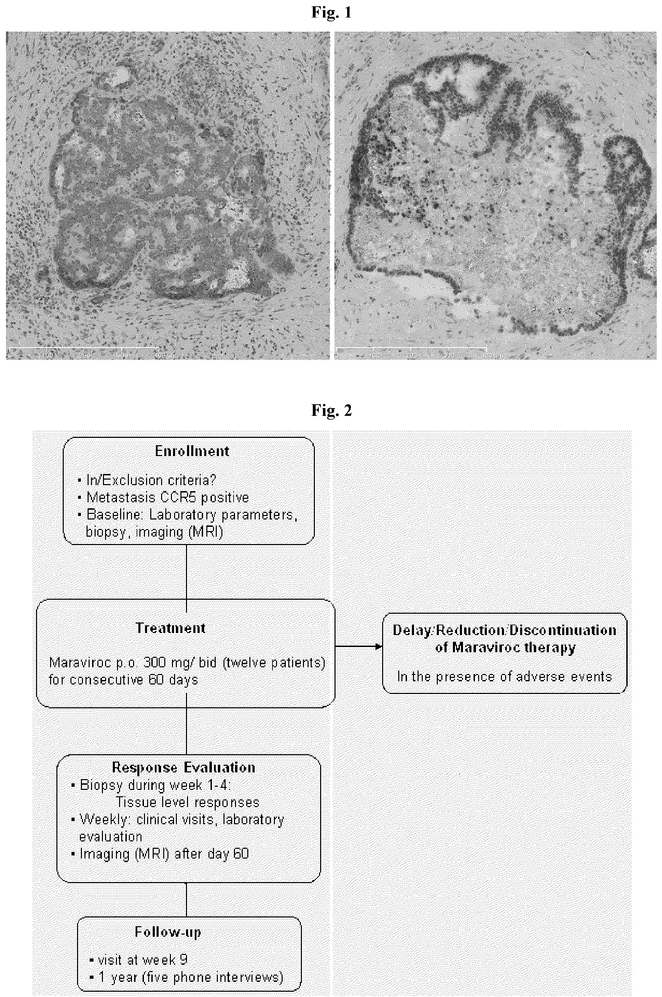

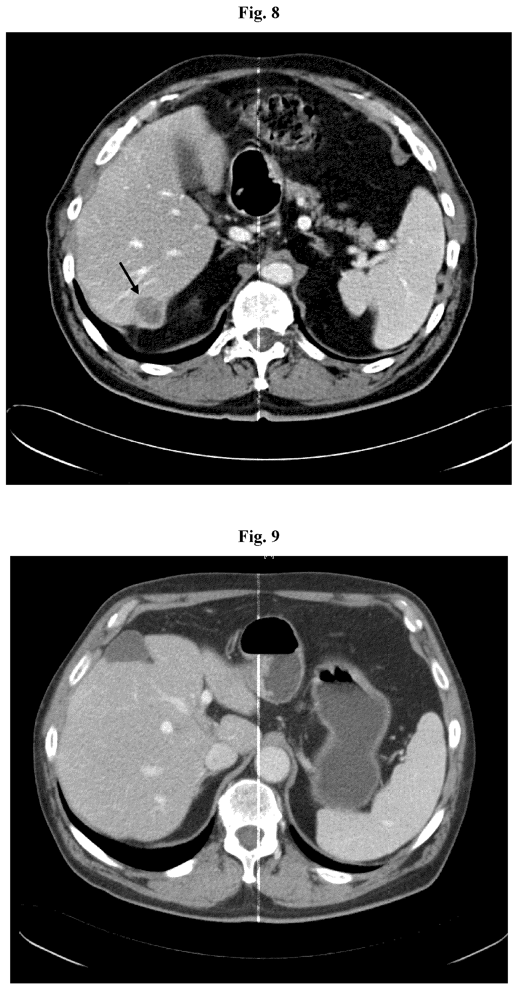

**Figure 1** *Apoptosis is induced by CCR5 blockade selectively in tumor cells (in an explant model)*
   Hemalaun counterstaining of a section from a colorectal cancer liver metastasis analyzed in an explant model setting. The left image shows the control tissue section (untreated) and the right image shows the treated counterpart from the facing section treated with maraviroc. Large areas of necrosis and only few remaining tumor cells can be seen.
**Figure 2** *Clinical trial overview showing diagnostic steps and biopsies in relation to treatment with Maraviroc.*
   A clinical phase I trial in advanced stage metastatic colorectal cancer patients after standard chemotherapy was initiated (EudraCT 2012-000861-18 and NCT01736813). Patients receive 300 mg / bid Maraviroc after a first pre-treatment biopsy and have a second biopsy of the same liver metastasis after treatment for 8-10 days.
**Figure 3** *Marked changes in key cytokines for tumor growth, chemotherapy resistance or angiogenesis are seen in patients treated with Maraviroc.*
   The light gray columns in the left side of each diagram show the cytokine concentrations at day 0 (pretreatment). The dark gray columns in the right side of each diagram show the cytokine concentrations at day 9 of treatment with maraviroc.
**Figure 4** *Reductions of proliferation activity (Ki67 percentage) are seen in patients treated with Maraviroc.*
   Percentages of (remaining) Ki67 positive tumor cells before and under treatment (8-10 days, second biopsy) from the tumor tissue. Error bars show s.e.m.
**Figure 5** *Clinical effects of Maraviroc treatment on liver metastases*
   Patients developed more central necrosis of liver metastases under treatment. The radiologically measured area of necrosis before and after therapy with CCR5 inhibition shows an increase in necrotic area (percentages of necrotic area for complete area of reference lesion). Error bars show s.e.m.
**Figure 6** *Immunohistochemical analysis of gastric cancer*
   CCR5 is expressed on gastric cancer cells.
**Figure 7** *Immunohistochemical analysis of hepatocellular cancer*
   CCR5 is expressed on hepatocellular cancer cells.
**Figure 8** *Clinical effects of Maraviroc treatment in combination with immunomodulation and radiation*
   CT scan of the abdomen. This patient was treated with Maraviroc 300 mg/bid and anti-PD-1 in combination with radiation (30 Gy) to the metastatic lesion. Responses were disappearance of the radiated metastatic lesion (see Figure 9) and reduction in overall tumor burden as well as significant tumor marker decrease. Arrow indicates reference metastatic lesion within the liver.
**Figure 9** *Clinical effects of Maraviroc treatment in combination with immunomodulation and radiation*
   CT scan of the abdomen. Complete disappearance of reference lesion (see Figure 8 for details, lesion indicated by arrow).

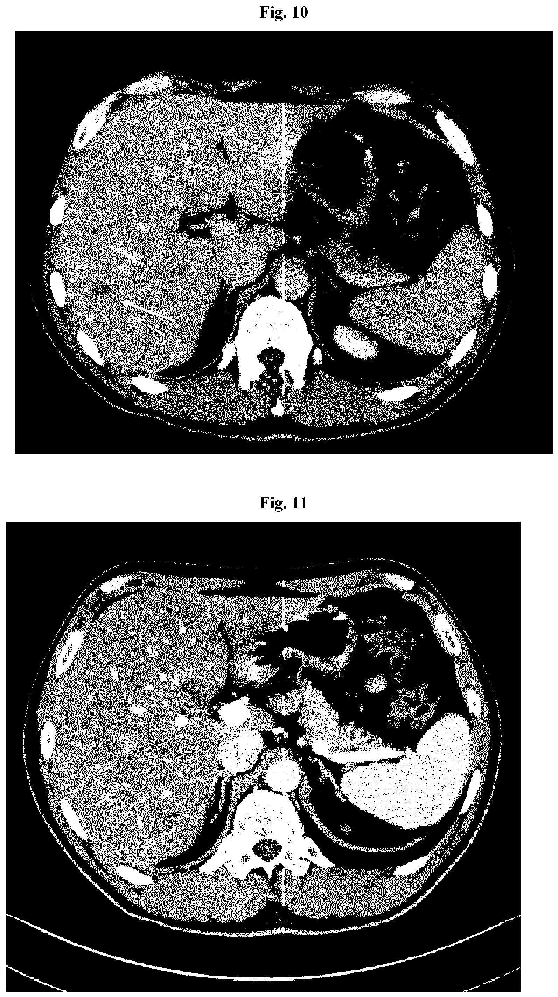

**Figure 10** *Clinical effects of Maraviroc treatment in combination with immunomodulation, chemotherapy and radiation*
   CT scan of the abdomen. This patient was treated again with oxaliplatinum-based therapy (after previous failure and after all standard of care treatments) and then with Maraviroc 300 mg/bid and anti-PD-1 in combination with radiation (20 Gy) to the metastatic lesion. Responses were disappearance of the radiated metastatic lesion (see Figure 11). White arrow indicates metastatic lesion within the liver.
**Figure 11** *Clinical effects of Maraviroc treatment in combination with immunomodulation, chemotherapy and radiation*
   CT scan of the abdomen. Complete disappearance of radiated lesion (see Figure 10 for details, lesion indicated by arrow).

### DETAILED DESCRIPTION OF THE INVENTION

### Definitions

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Several documents (for example: patents, patent applications, scientific publications, manufacturer's specifications, instructions, GenBank Accession Number sequence submissions etc.) are cited throughout the text of this specification. Nothing herein is to be construed as an admission that the invention is not entitled to antedate such disclosure by virtue of prior invention. In the event of a conflict between the definitions or teachings of documents cited herein and definitions or teachings recited in the present specification, the text of the present specification takes precedence.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "150 mg to 600 mg" should be interpreted to include not only the explicitly recited values of 150 mg to 600 mg, but also include individual values and sub-ranges within the indicated range. Thus, included in this numerical range are individual values such as 150, 160, 170, 180, 190, ... 580, 590, 600 mg and sub-ranges such as from 150 to 200, 150 to 250, 250 to 300, 350 to 600, etc. This same principle applies to ranges reciting only one numerical value. Furthermore, such an interpretation should apply regardless of the breadth of the range or the characteristics being described.

The term "about" when used in connection with a numerical value is meant to encompass numerical values within a range having a lower limit that is 5% smaller than the indicated numerical value and having an upper limit that is 5% larger than the indicated numerical value.

"bid" is the abbreviation for the Latin expression *"bis in die",* which means twice daily.

In the context of the present application, the term "CCR5 antagonist" relates to a compound that inhibits at least one biological activity of CCR5. Preferably it inhibits the interaction of the receptor CCR5 with its ligands and the subsequent activation of the receptor. As used herein, a "CCR5 antagonist" especially inhibits the interaction of the receptor CCR5 with ligands CCL5, CCL3 and/or CCL4. Tests for determining binding between CCR5 and its ligands as well as determining the inhibition of such binding have been described by Dorr P. et al., 2005 and Haworth B. et al., 2007. In the context of the present disclosure, a substance is considered to be a "CCR5 antagonist", if it exhibits at least 1% (e.g. at least 5%, at least 10%, at least 20%, at least 30%, at least 40%, or at least 50%) of the blocking activity of maraviroc on the interaction between CCR5 and one of its ligands, preferably the interaction between CCR5 and CCL5. In the context of the present application, a "CCR5 antagonist" preferably exhibits no intrinsic CCR5 agonistic effect.

MIF is the abbreviation for *macrophage migration inhibitory factor.* The UNIPROT number for human MIF is P14174.

IL-8 (interleukin 8) is a chemokine produced by macrophages and other cell types such as epithelial cells, airway smooth muscle cells and endothelial cells. The UNIPROT number for human IL-8 is P10145.

HGF is the abbreviation for *hepatocyte growth factor.* The UNIPROT number for human HGF is P14210.

SCGF is the abbreviation for *stem cell growth factor.* This growth factor is also known as C-type lectin domain family 11 member A. The UNIPROT number for human SCGF is Q9Y240.

IL-1ra is the abbreviation for *interleukin-1 receptor antagonist.* The UNIPROT number for human IL-1ra is P18510.

MCP-1 is the abbreviation for *monocyte chemotactic protein-1.* This cytokine is also known as chemokine (C-C motif) ligand 2 (CCL2) and as small inducible cytokine A2. The UNIPROT number for human MCP-1 is P13500.

VEGF is the abbreviation for *vascular endothelial growth factor.* VEGF is a sub-family of growth factors comprising VEGF-A, placenta growth factor (PGF), VEGF-B, VEGF-C and VEGF-D. If not explicitly specified otherwise, the term "VEGF" refers to VEGF-A in the context of the present application. The UNIPROT number for human VEGF-A is P15692.

VCAM-1 is the abbreviation for *vascular cell adhesion molecule 1.* This cell adhesion molecule is also known as vascular cell adhesion protein 1 or cluster of differentiation 106 (CD106). The UNIPROT number for human VCAM-1 is P19320.

As used herein, "treat", "treating" or "treatment" of a disease or disorder means accomplishing one or more of the following: (a) reducing the severity and/or duration of the disorder; (b) limiting or preventing development of symptoms characteristic of the disorder(s) being treated; (c) inhibiting worsening of symptoms characteristic of the disorder(s) being treated; (d) limiting or preventing recurrence of the disorder(s) in patients that have previously had the disorder(s); and (e) limiting or preventing recurrence of symptoms in patients that were previously symptomatic for the disorder(s).

The present invention particularly relates to the treatment of cancer. As used herein, "treatment of cancer" means accomplishing one or more of the following: (i) tumor growth inhibition and/or tumor cell death; (ii) reduction of tumor marker(s); (iii) reduction of tumor lesions and metastases; (iv) reduction of tumor burden as evidenced by imaging studies (e.g. CT, MRI, PET etc.); and (v) reduction of tumor burden as evidenced by clinical appraisal or self-report by the patient

As used herein, the expressions "is for administration" and "is to be administered" have the same meaning as "is prepared to be administered". In other words, the statement that an active compound "is for administration" has to be understood in that said active compound has been formulated and made up into doses so that said active compound is in a state capable of exerting its therapeutic activity.

The terms "therapeutically effective amount" or "therapeutic amount" are intended to mean that amount of a drug or pharmaceutical agent that will elicit the biological or medical response of a tissue, a system, animal or human that is being sought by a researcher, veterinarian, medical doctor or other clinician. The dosage regimen utilizing a CCR5 antagonist as described herein can be selected by the skilled practitioner in accordance with a variety of factors including type, species, age, weight, body mass index, sex and medical condition of the patient; the severity of the condition to be treated; the potency of the compound chosen to be administered; the route of administration; the purpose of the administration; and the renal and hepatic function of the patient.

The terms "subject" and "patient" are used interchangeably herein.

As used herein, a "patient" means any mammal or bird that/who may benefit from a treatment with the CCR5 antagonists described herein. Preferably, a "patient" is selected from the group consisting of laboratory animals (e.g. mouse or rat), domestic animals (including e.g. guinea pig, rabbit, chicken, turkey, pig, sheep, goat, camel, cow, horse, donkey, cat, or dog), or primates including chimpanzees and human beings. It is particularly preferred that the "patient" is a human being.

"Pharmaceutically acceptable" means approved by a regulatory agency of the Federal or a state government or listed in the U.S. Pharmacopeia (United States Pharmacopeia-33/National Formulary-28 Reissue, published by the United States Pharmacopeial Convention, Inc., Rockville Md., publication date: April 2010) or other generally recognized pharmacopeia for use in animals, and more particularly in humans.

### Embodiments of the Invention

The present invention is defined by the subject-matter of the appended claims.

The present invention will now be further described. In the following passages different aspects of the invention are defined in more detail.

As discussed above, the present invention provides a CCR5 antagonist for use in the treatment of metastatic colorectal cancer in a subject receiving immunomodulatory therapy with anti-PD-1, wherein the CCR5 antagonist is Maraviroc or [3H]maraviroc. A common pattern in biology is the "receptor - ligand" dualism. A molecule acts as a "key" (ligand) and triggers effects in a second molecule, the "lock" (receptor). Many molecules are present in all life forms with distinct receptors and ligands being present in all embodiments of life. Especially the immune system is tightly regulated via specific receptors. A subclass of these receptors is of key relevance in processes of migration: the chemokines. These chemokine receptors are typically found on migrating cells like lymphocytes or macrophages. Of these chemokine receptors, the CC chemokine receptor 5 (CCR5) belongs to the superfamily of 7-transmembrane spanning G-protein coupled receptors (GPCR) (Oppermann M. et al., 2004). The physiological role of CCR5 lies within the coordination and regulation of T cells, macrophages and immature dendritic cells. Therefore the receptor is expressed on effector T cells, macrophages, and immature dendritic cells (Oppermann et al., 2004). The receptor has three known ligands: CC chemokine ligand 5 (CCL5, syn. RANTES), CCL3 and CCL4 bind to CCR5 that is followed by activation of the receptor. The main functional role of CCR5 is chemotaxis, the active recruitment of immune cells into inflammatory sites including tumors upon chemokine attraction (directed migration of lymphocytes) (Musha H. et al., 2005; Ohtani H., et al. 2007). A process known as polarization reorganizes the intracellular cytoskeleton, resulting in the formation of a front end (leading edge) as well as a back end (trailing edge), which supports cell migration out of the vasculature (Wong M.M. et al., 2003). Besides its role in cell recruitment and polarization, CCR5 stimulation is involved in immunologic effector functions including activation and differentiation of T cells as well as cytokine production (Oppermann et al., 2004; Wong et al., 2003). Furthermore, CCR5 is a co-receptor for the infection with the human immunodeficiency virus (HIV) (Dorr P. et al., 2005; Berger E.A. et al., 1999). More precisely, CCR5 is a co-receptor for HIV entry into the host cells. The available antagonists of CCR5 are cell entry inhibitors for HIV and have (potential) therapeutic applications in the treatment of HIV infections.

In cancer patients the role for CCR5 was unclear. Recent work by our group (see section Examples) has found a surprising role of CCR5 in colorectal cancer and in other cancer entities. Immunological processes influence the clinical course of cancer, for example of colorectal cancer (CRC). Analyzing human CRC liver metastases and especially the invasive margin, we now found that multiple levels of immune evasion mechanisms protect the tumor from the host's immune system. The host's immune system produces CCL5, an important ligand for CCR5. CCL5 produced by these T cells stimulates tumor cell proliferation and invasive behavior of tumor cells via CCR5 on tumor cells. We therefore initiated a phase I trial (see section Examples below) with a CCR5 antagonist in CRC patients with liver metastases that validates the predicted modification of the tumor-promoting inflammation efficacy of this treatment on the tissue level and also shows clinical efficacy in humans. As detailed in the Example section below, the clinical effects of CCR5 blockade could be dramatically improved by adding chemotherapy, immunotherapy, radiation or combinations of these and other therapies to the CCR5 blockade. When treating treatment-refractory patients with these new combinations, unexpected dramatic reductions of the tumor burden could be induced and patient care could be improved significantly.

For clinical use, CCR5 antagonists have to have certain properties. CCR5 antagonists or CCR5 receptor antagonists are specific compounds or molecules that antagonize the CCR5 receptor, i.e. they block the interaction of CCR5 with its ligands, such as CCL5. Due to the location of CCR5 receptors at the cell surface, both large and small molecules or compounds have the potential to interfere with ligand binding on human cells. It is vital to recognize that according to the invention, a "CCR5 antagonist" or "CCR5 inhibitor" preferably exhibits no or no significant intrinsic CCR5 agonistic effect and does not lead to relevant activation of the receptor. Preferably the CCR5 antagonist or inhibitor is a molecule that is orally bioavailable, i.e. which can be ingested by patients. In addition, therapies that lead to a decreased production of the CCR5 receptor (preferably on the tumor cells) are also regarded as a "CCR5 antagonist" or "CCR5 inhibitor" according to this invention.

In a first aspect the present invention is directed to a CCR5 antagonist for use in the treatment of metastatic colorectal cancer in a subject receiving immunomodulatory therapy with anti-PD-1, wherein said CCR5 antagonist is Maraviroc or [3H]maraviroc.

Described is a method for the treatment of cancer, comprising the step:
administering a therapeutic amount of a CCR5 antagonist to a subject in need thereof, wherein said subject receives at least one further cancer therapy.

In other words, the first aspect relates to a combination therapy for the treatment of cancer. The administration of CCR5 (which is considered to be a first anti-cancer therapy within the context of this application) is combined with a second anti-cancer therapy.

Described herein are cancers to be treated selected from the group consisting of primary pancreatic cancer, metastatic pancreatic cancer, primary colorectal cancer, metastatic colorectal cancer, primary ovarial cancer, metastatic ovarial cancer, primary renal cell cancer, metastatic renal cell cancer, primary stomach cancer, metastatic stomach cancer, primary prostate cancer, metastatic prostate cancer, primary breast cancer, metastatic breast cancer, primary hepatocellular cancer, metastatic hepatocellular cancer, primary lung cancer, metastatic lung cancer, primary head and neck cancer, metastatic head and neck cancer, primary gastric cancer, metastatic gastric cancer, primary esophageal cancer, and metastatic esophageal cancer.

Described herein is the treatment of cancers in very early stages, from pre-malignant lesions, through primary tumors (non-metastatic) to metastatic stages (primary tumors with lymph node involvement, vessel invasion, lymph vessel invasion, distant organ metastases of all sites). Described herein is the treatment of a cancer that only appears as metastasis without a designated primary tumor (cancer of unknown primary). Further described herein is the treatment of a metastatic primary tumor that has been removed surgically and an adjuvant treatment is needed to treat (suspected) microscopic remaining tumor burden, which then is performed as a combination treatment with a CCR5 antagonist. The cancer to be treated according to the present invention is metastatic colorectal cancer.

In another embodiment of the first aspect, the subject receives at least one further anti-cancer therapy selected from the group consisting of chemotherapy, radiation therapy, vaccination, immunotherapy, stem cell therapy, anti-hormonal therapy, local ablation of metastases or tumors by physical intervention, and combinations of any of the above. An exemplary combination of anti-cancer therapies comprises the administration of a CCR5 antagonist with chemotherapy and radiation therapy.

In those embodiments, in which the anti-cancer-therapy is chemotherapy, the chemotherapy may comprise the administration of an alkylating agent, an antimetabolite, folinic acid, a folate antagonist, a mitotic inhibitor, an anthracyclin, a topoisomerase inhibitor, an antibody, a signal transduction inhibitor, an inhibitor of angiogenesis, and an inhibitor of histone deacetylase.

The alkylating agent can be selected from the group consisting of bendamustin, busulfan, carboplatin, carmustin, cisplatin, oxaliplatin, cyclophosphamid, mitomycin, and treosulfan. The antimetabolite can be selected from the group consisting of 5-fluorouracil, capecitabin, cytarabin, gemcitabin, mercaptopurin, and deoxyglucose. The folate antagonist can be selected from the group consisting of methotrexate and pemetrexed. The mitotic inhibitor can be selected from the group consisting of taxanes and vinca-alcaloids. Taxanes suitable for use in the present invention include for example cabzitaxel, docetaxel, or paclitaxel. Paclitaxel can be administered as nanoparticle albumin bound paclitaxel (nab-paclitaxel, commercially available from Celgene Corp. under the trade name Abraxane). The anthracyclin can be selected from the group consisting of bleomycin, doxorubicin, mitoxantron, and epirubicin. The topoisomerase inhibitor can be selected from the group consisting of campthotecin derivatives and podophyllin derivatives. Campthotecin-derivatives suitable for use in the present invention include for example irinotecan and topotecan. The antibody can be selected from the group consisting of bevacizumab (Avastin^{®}, Roche), cetuximab (Erbitux^{®}, Bristol-Myers Squibb and Merck KGaA), panitumumab (Vectibix^{®}, Amgen), ipilimumab, nuvolimumab, tremelimumab, anti-OX40-antibodies, catumaxomab, and anti-CD40L antibodies. The signal transduction inhibitor can be selected from the group consisting of axatinib, crizotinib, erlotinib, sunitinib, sorafenib, everolimus, imatinib, lapatinib, pazopanib, temsirolimus, and vemurafenib. An inhibitor of angiogenesis suitable for use in the present invention is aflibercept. An inhibitor of histone deacetylase suitable for use in the present invention is vorinostat.

In one embodiment of the first aspect, the at least one further anti-cancer therapy is a chemotherapy comprising the administration of one or more (e.g., 1, 2, 3, 4, 5, 6, or 7) of the following substances: folinic acid, carboplatin, oxaliplatin, 5-fluorouracil, gemcitabin, paclitaxel or bevacizumab.

In those embodiments, in which the anti-cancer-therapy is radiation therapy, said radiation therapy may include all forms of radiation, including heavy ion. Radiation therapy as a combination therapy with a CCR5 antagonist does not necessarily need to be within a given therapeutic intensity, single doses as low as 1 Gy can have beneficial effects in combination with CCR5 inhibition. A single dose may have a low or high intensity. A low intensity is in the range of 1-30 Gy, i.e. 1, 2, 3, 4, 5, 6, 7, 8, 9, 10, 15, 20, 25, 26, 27, 28, 29, or 30 Gy.
A high intensity is within the range of 31-100 Gy, i.e. 31, 32, 33, 34, 35, 36, 37, 38, 39, 40, 45, 50, 55, 60, 65, 70, 75, 80, 85, 90, 95, or 100 Gy. Accordingly, a single dose may have an intensity of 1, 2, 3, 4, 5, 6, 7, 8, 9, 10, 15, 20, 25, 30, 35, 40, 45, 50, 55, 60, 65, 70, 75, 80, 85, 90, 95, or 100 Gy. The intensity of a single dose may thus be in the range of 1-80 Gy, 2-80 Gy, 3-80 Gy, 4-80 Gy, 5-80 Gy, 6-80 Gy, 7-80 Gy, 8-80 Gy, 9-80 Gy, 10-80 Gy, 15-80 Gy, 20-80 Gy, 25-80 Gy, 30-80 Gy, 35-80 Gy, 40-80 Gy, 45-80 Gy, 50-80 Gy, 55-80 Gy, 60-80 Gy, 65-80 Gy, 70-80 Gy, or75-80 Gy, The intensity of a single dose may also be in the range of 1-30 Gy, 2-30 Gy, 3-30 Gy, 4-30 Gy, 5-30 Gy, 6-30 Gy, 7-30 Gy, 8-30 Gy, 9-30Gy, 10-30 Gy, 15-30 Gy, 20-30 Gy, or 25-30 Gy. The intensity of a single dose may also be in the range of 31-40 Gy, 31-45 Gy, 31-50 Gy, 31-55 Gy, 31-60 Gy, 31-65 Gy, 31-70 Gy, 31-75 Gy, 31-80 Gy, 31-85 Gy, 31-90 Gy, 31-95 Gy, or 31-100 Gy.

Described herein is an anti-cancer-therapy that is an immunotherapy, wherein said immunotherapy includes all interventions using immunological mechanisms (i.e. adoptive T cell transfer, chimeric antigen receptor therapy, antibodies against tumor targets, anti-PD-1 therapy, etc.).

Described herein is an anti-cancer-therapy that is an immunomodulatory therapy, wherein said immunomodulatory therapy includes all interventions aimed at modulating (activating or inhibiting) specific elements of the immune system of the host or the tumor microenvironment (e.g. CTLA-4 blockade, anti-PD-L1 therapy, macrophage modulation, CD40L agonists etc.). Described herein is the combination of a CCR5 antagonist with an immunomodulatory drug selected from the group consisting of anti-PD-1, anti-PD-L1, anti-CD40 (Agonist), CD40-Ligand, anti-GM-CSF, anti-CSF-1R and anti-CTLA-4.

In those embodiments, in which the anti-cancer-therapy is stem cell therapy, said stem cell therapy can be a treatment with interferon alpha or treatment with neutralizing antibodies against stem cell (growth) factors.

In those embodiments, in which the anti-cancer-therapy is anti-hormonal therapy, said anti-hormonal therapy may comprise androgen deprivation (anti-androgens or GnRH-antagonists or androgen-synthesis inhibitors or androgen uptake inhibitors), estrogen-deprivation (conversion inhibitors, aromatase inhibition, E2 blockade), progesterone-deprivation and others (e.g. abarelix, abriateronacetate, anastrozole, bicalutamid, buserelin, cyproteronacetate, degarelix, exemestan, flutamid, faslodex, goserelin, histrel, letrozole, leuprorelin, medroxyprogesterone, megestrol, tamoxifen, toremifen, triptorelin and others).

In those embodiments, in which the anti-cancer-therapy is a local ablation of metastases or tumors by physical intervention, this local ablation may comprise one or more of the following treatments: heat or cold, high frequency ablation, instillation of chemicals, radioactively labeled particles, and surgical intervention.

In further embodiments, the anti-cancer-therapy is an immunomodulatory therapy and a radiation therapy. In particular embodiments, the CCR5 antagonist is combined with an immunomodulatory drug and radiation therapy. In particular, the CCR5 antagonist is combined with an immunomodulatory drug selected from the group consisting of anti-PD-1, anti-PD-L1, anti-CD40 (Agonist), CD40-Ligand, anti-GM-CSF, anti-CSF-1R and anti-CTLA-4, and radiation therapy consisting of low dose radiation with 2-30 Gy doses or high dose radiation with 31-100 Gy radiation .

In further embodiments, the anti-cancer-therapy is a chemotherapy, and a radiation therapy. In particular embodiments, the CCR5 antagonist is combined with anti-PD-1, chemotherapy and radiation therapy. Described is also the combination of a CCR5 antagonist with an immunomodulatory drug selected from the group consisting of anti-PD-1, anti-PD-L1, anti-CD40 (Agonist), CD40-Ligand, anti-GM-CSF, anti-CSF-1R and anti-CTLA-4, the chemotherapy consists of platinum-based therapies (e.g. FOLFOX), gemcitabine, or taxanes (e.g. paclitaxel), and the radiation therapy consisting of low dose radiation with 2-30 Gy doses or high dose radiation with 31-100 Gy radiation .

In preferred embodiments of the first aspect, wherein the treatment modalities are used sequentially or concurrent.

Described are CCR5 antagonists selected from the group consisting of Maraviroc (Pfizer), Vicriviroc (Schering-Plough), Aplaviroc (GlaxoSmithKline(GSK)), ancriviroc, [3H]maraviroc, [3H]ancriviroc, CCL7, TAK-779, E913, TAK-652, TAK-220, and vMIP-II. Only maraviroc and [3H]maraviroc are CCR5 antagonists of the present invention.

Maraviroc is a potent, orally bioavailable CCR5 antagonist which selectively blocks the interaction of the receptor CCR5 with its ligands. IUPAC name / INN: 4,4-difluoro-*N*-{(1*S*)-3-[3-(3-isopropyl- 5-methyl-4*H*-1,2,4-triazol-4-yl)-8-azabicyclo[3.2.1]oct-8-yl]-1-phenylpropyl}cyclohexanecarboxamide. Maraviroc (brand-named Selzentry, or Celsentri outside the U. S.) is an antiretroviral drug in the CCR5 receptor antagonist class that has received FDA approval in 2006 (extension of EMEA 2012) and has been used since then in the treatment of HIV infection. Specifically, maraviroc is a negative allosteric modulator of the CCR5 receptor. The chemokine receptor CCR5 is an essential co-receptor for most HIV strains and necessary for the entry process of the virus into the host cell. The drug binds to CCR5, thereby blocking the HIV protein gp120 from associating with the receptor. HIV is then unable to enter human macrophages and T-cells.

In some embodiments of the first aspect, more than one CCR5 antagonist is used; e.g. two, three, four or more different CCR5 antagonists can be used in combination.

In further embodiments of the first aspect, the CCR5 antagonist is to be administered in an amount of between about 150 mg and about 600 mg once or twice a day, e.g. about 150 mg once per day, or about 150 mg twice daily, or about 300 mg once per day, or about 300 mg twice daily, or about 600 mg once per day, or about 600 mg twice daily. For example, the above doses can be administered for an unlimited amount of time with doses being potentially adapted to medical needs. An exemplary treatment regimen can also consist of three weeks of CCR5 inhibition and a pause for one week, the four week cycle being repeated.

In one embodiment of the first aspect, the CCR5 antagonist is to be administered orally, intravenously or topically.

Combination therapies described herein are set forth below:
For example for colorectal cancer:
- the chemotherapeutic agents are selected from oxaliplatinum, irinotecan, 5-FU, capecitabin, mitomycine, gemcitabine, paclitaxel (such as nab-paclitaxel / abraxane^{®}) or combinations thereof,
- the antibody/antibodies is/are preferably selected from cetuximab, bevacizumab, panitumumab, ipilimumab, tremelimumab, anti-OX40-antibodies, catumaxomab, anti-CD40L antibodies or others or combinations thereof.
- The chemotherapeutic regimen that is selected for combination therapy with a CCR5 antagonist can be selected from the previous treatments the patient already received and is preferably a treatment regimen that was successful for a given time period in this patient.
- Radiation therapy can be of any intensity (e.g. 1 to 80 Gy, i.e. 1, 2, 3, 4, 5, 6, 7, 8, 9, 10, 15, 20, 25, 30, 35, 40, 45, 50, 55, 60, 65, 70, 75, 80, 85, 90, 95, or 100 Gy) given the combination with CCR5 inhibition and (subsequent) chemotherapy.
- In one embodiment (FOLFOX4 plus Bevacizumab and Maraviroc) oxaliplatinum (dose 85 mg/m² body surface area, as infusion for 2-6h on day 1), folinic acid (dose 200 mg/m² body surface area, as i.v. bolus on day 1 and 2), 5-Fluorouracil (dose 400 mg/m² body surface area, as i.v. bolus on day 1 and 2), 5-Fluorouracil (dose 600 mg/m² body surface area, as infusion over 22h on day 1 to 2) and bevacizumab (5 mg/kg body weight as infusion over 30 to 60 minutes on day 1) are given and the whole regimen is repeated on day 15 with Maraviroc (dose 300 mg/bid) given daily. Alternatively, other chemotherapy regimens like FOLFOX6 or similar ones that are known to persons skilled in the art can be used in combination with Maraviroc.
- In one embodiment (FOLFIRI plus Bevacizumab plus Maraviroc) irinotecan (dose 180 mg/m² body surface area, as infusion for 90 minutes on day 1), folinic acid (dose 200 mg/m² body surface area, as i.v. bolus on day 1 and 2), 5-Fluorouracil (dose 400 mg/m² body surface area, as i.v. bolus on day 1 and 2), 5-Fluorouracil (dose 600 mg/m² body surface area, as infusion over 22h on day 1 to 2) and bevacizumab (5 mg/kg body weight as infusion over 30 to 60 minutes on day 1) are given and the whole regimen is repeated on day 15 with Maraviroc (dose 300 mg/bid) given daily.

For example for pancreatic cancer:
- the chemotherapeutic agents are selected from gemcitabine, paclitaxel (such as nab-paclitaxel / Abraxane^{®}), oxaliplatinum, irinotecan or combinations thereof
- in one embodiment gemcitabin and paxlitaxel are combined with CCR5 inhibition, e.g. in Gemcitabin given at day 1, 8, 15 (repetition at day 28) at 100 mg/m² body surface area, while nab-Paclitaxel is given every three weeks and Maraviroc at 300 mg/bid continuously.

In a second aspect the present invention is directed to a CCR5 antagonist for use in the treatment of metastases of colorectal carcinoma by inducing tumor cell death, wherein said CCR5 antagonist is Maraviroc or [3H]maraviroc.

Described is a method for the treatment of metastases of colorectal carcinoma, comprising the step:
administering a therapeutic amount of a CCR5 antagonist to a subject in need thereof.

In other words, the second aspect of the present invention relates to a monotherapy for the treatment of metastases of colorectal carcinoma, i.e. one or more CCR5 antagonists are administered to the subject but the subject does not receive any further anti-cancer therapy, especially no further chemotherapy, while receiving the CCR5 antagonist(s).

In one embodiment of the second aspect, the metastases are selected from the group consisting of liver metastases, lung metastases, bone metastases, pancreas metastases, lymph node metastases, and peritoneal metastases.

Described herein are CCR5 antagonists selected from the group consisting of Maraviroc (Pfizer), Vicriviroc (Schering-Plough), Aplaviroc (GlaxoSmithKline(GSK)), ancriviroc, [3H]maraviroc, [3H]ancriviroc, CCL7, TAK-779, E913, TAK-652, TAK-220, and vMIP-II. Only maraviroc and [3H]maraviroc are CCR5 antagonists of the present invention.

In some embodiments of the second aspect, more than one CCR5 antagonist is used; e.g. two, three, four or more different CCR5 antagonists can be used in combination.

In further embodiments of the second aspect, the CCR5 antagonist is to be administered in an amount of between about 150 mg and about 600 mg once or twice a day, e.g. about 150 mg once per day, or about 150 mg twice daily, or about 300 mg once per day, or about 300 mg twice daily, or about 600 mg once per day, or about 600 mg twice daily. For example, the above doses can be administered for an unlimited amount of time with doses being potentially adapted to medical needs. An exemplary treatment regimen can also consist of three weeks of CCR5 inhibition and a pause for one week, the four week cycle being repeated.

In one embodiment of the second aspect, the CCR5 antagonist is to be administered orally, intravenously or topically.

Further described herein, and not forming part of the present invention, is a method for determining whether a subject benefits from a therapeutic treatment of cancer by a CCR5 antagonist, said method comprising the steps of:
comparing the level of at least one cytokine in a test sample isolated from a subject undergoing therapeutic treatment of cancer by a CCR5 antagonist to the level of the same cytokine in a test sample isolated from the same subject prior to said therapeutic treatment (= pre-treatment level), wherein a reduction of the level of said at least one cytokine demonstrates that the subject benefits from said therapeutic treatment.

The method may additionally comprise the following step:
continuing treatment with the CCR5 antagonist, if it has been determined in the first step of the method that the subject benefits from said therapeutic treatment.

The method may additionally compriss the following step:
discontinuing treatment with the CCR5 antagonist, if it has been determined in the first step of the method that the subject does not benefit from said therapeutic treatment.

The cytokine may be selected from the group consisting of MIF, IL-8, HGF, SCGF, IL-1ra, MCP-1, VEGF and VCAM-1.

A reduction of the level of said at least one cytokine by 10% or more (e.g. 20% or more, 30% or more, 40% or more, 50% or more, 60% or more, 70% or more, 80% or more 90% or more) as compared to the pre-treatment level of said cytokine may demonstrate that the subject benefits from said therapeutic treatment.

A reduction of the level of MIF by 10% or more (e.g. 20% or more, 30% or more, 40% or more, 50% or more, 60% or more, 70% or more, 80% or more 90% or more) as compared to the pre-treatment level of MIF demonstrates that the subject benefits from said therapeutic treatment.

A reduction of the level of IL-8 by 10% or more (e.g. 20% or more, 30% or more, 40% or more, 50% or more, 60% or more, 70% or more, 80% or more 90% or more) as compared to the pre-treatment level of IL-8 demonstrates that the subject benefits from said therapeutic treatment.

A reduction of the level of HGF by 10% or more (e.g. 20% or more, 30% or more, 40% or more, 50% or more, 60% or more, 70% or more, 80% or more 90% or more) as compared to the pre-treatment level of HGF demonstrates that the subject benefits from said therapeutic treatment.

A reduction of the level of SCGF by 10% or more (e.g. 20% or more, 30% or more, 40% or more, 50% or more, 60% or more, 70% or more, 80% or more 90% or more) as compared to the pre-treatment level of SCGF demonstrates that the subject benefits from said therapeutic treatment.

A reduction of the level of IL-1ra by 10% or more (e.g. 20% or more, 30% or more, 40% or more, 50% or more, 60% or more, 70% or more, 80% or more 90% or more) as compared to the pre-treatment level of IL-1ra demonstrates that the subject benefits from said therapeutic treatment.

A reduction of the level of MCP-1 by 10% or more (e.g. 20% or more, 30% or more, 40% or more, 50% or more, 60% or more, 70% or more, 80% or more 90% or more) as compared to the pre-treatment level of MCP-1 demonstrates that the subject benefits from said therapeutic treatment.

A reduction of the level of VEGF by 10% or more (e.g. 20% or more, 30% or more, 40% or more, 50% or more, 60% or more, 70% or more, 80% or more 90% or more) as compared to the pre-treatment level of VEGF demonstrates that the subject benefits from said therapeutic treatment.

A reduction of the level of VCAM-1 by 10% or more (e.g. 20% or more, 30% or more, 40% or more, 50% or more, 60% or more, 70% or more, 80% or more 90% or more) as compared to the pre-treatment level of VCAM-1 demonstrates that the subject benefits from said therapeutic treatment.

In the described method, the cancer is selected from the group consisting of primary pancreatic cancer, metastatic pancreatic cancer, primary colorectal cancer, metastatic colorectal cancer, primary ovarial cancer, metastatic ovarial cancer, primary renal cell cancer, metastatic renal cell cancer, primary stomach cancer, metastatic stomach cancer, primary prostate cancer, metastatic prostate cancer, primary breast cancer, metastatic breast cancer, primary hepatocellular cancer, metastatic hepatocellular cancer, primary lung cancer, metastatic lung cancer, primary head and neck cancer, metastatic head and neck cancer, primary gastric cancer, metastatic gastric cancer, primary esophageal cancer, and metastatic esophageal cancer.

In the described method, the cancer can be in very early stages, from pre-malignant lesions, through primary tumors (non-metastatic) to metastatic stages (primary tumors with lymph node involvement, vessel invasion, lymph vessel invasion, distant organ metastases of all sites). In the described method, the cancer only appears as metastasis without a designated primary tumor (cancer of unknown primary). In the described method, the metastatic primary tumor has been removed surgically and the cancer comprises a (suspected) microscopic remaining tumor burden. The cancer to be treated according to the present invention is metastatic colorectal cancer.

In the described method, the sample is a tissue sample, in particular a tissue sample from the cancerous tissue.

In the described method, the CCR5 antagonist is selected from the group consisting of Maraviroc (Pfizer), Vicriviroc (Schering-Plough), Aplaviroc (GlaxoSmithKline(GSK)), ancriviroc, [3H]maraviroc, [3H]ancriviroc, CCL7, TAK-779, E913, TAK-652, TAK-220, and vMIP-II. Only maraviroc and [3H]maraviroc are CCR5 antagonists of the present invention.

In the described method, the therapeutic treatment can be a monotherapy with a CCR5 antagonist as explained in greater detail above, when discussing the second aspect of the invention.

In the described method, the therapeutic treatment can be a combination therapy with a CCR5 antagonist and another anti-cancer therapy as explained in greater detail above, when discussing the first aspect of the invention.

Further described is a method, not forming part of the present invention, for determining whether a subject will benefit from a therapeutic treatment of cancer by a CCR5 antagonist, said method comprising the steps of:
(a) obtaining a biopsy sample of tumor tissue from a subject;
(b) cultivating a first portion of said biopsy sample in the presence of a CCR5 antagonist;
(c) cultivating a second portion of said biopsy sample in the absence of a CCR5 antagonist;
(d) determining the level of at least one cytokine in the portion cultivated in step (b);
(e) determining the level of the same at least one cytokine in the portion cultivated in step (c);
(f) comparing the level of said at least one cytokine determined in step (d) to the level of said at least one cytokine determined in step (e), wherein a reduced level in step (d) as compared to step (e) indicates that the subject will benefit from a therapeutic treatment of cancer by a CCR5 antagonist.

In the described method, the cytokine is selected from the group consisting of MIF, IL-8, HGF, SCGF, IL-1ra, MCP-1, VEGF and VCAM-1.

In the described method, a reduction of the level of said at least one cytokine determined in step (d) by 10% or more (e.g. 20% or more, 30% or more, 40% or more, 50% or more, 60% or more, 70% or more, 80% or more 90% or more) as compared to the level of said at least one cytokine determined in step (e) demonstrates that the subject will benefit from said therapeutic treatment.

In the described method, a reduction of the level of MIF determined in step (d) by 10% or more (e.g. 20% or more, 30% or more, 40% or more, 50% or more, 60% or more, 70% or more, 80% or more 90% or more) as compared to the level of MIF determined in step (e) demonstrates that the subject will benefit from said therapeutic treatment.

In the described method, a reduction of the level of IL-8 determined in step (d) by 10% or more (e.g. 20% or more, 30% or more, 40% or more, 50% or more, 60% or more, 70% or more, 80% or more 90% or more) as compared to the level of IL-8 determined in step (e) demonstrates that the subject will benefit from said therapeutic treatment.

In the described method, a reduction of the level of HGF determined in step (d) by 10% or more (e.g. 20% or more, 30% or more, 40% or more, 50% or more, 60% or more, 70% or more, 80% or more 90% or more) as compared to the level of HGF determined in step (e) demonstrates that the subject will benefit from said therapeutic treatment.

In the described method, a reduction of the level of SCGF determined in step (d) by 10% or more (e.g. 20% or more, 30% or more, 40% or more, 50% or more, 60% or more, 70% or more, 80% or more 90% or more) as compared to the level of SCGF determined in step (e) demonstrates that the subject will benefit from said therapeutic treatment.

In the described method, a reduction of the level of IL-1ra determined in step (d) by 10% or more (e.g. 20% or more, 30% or more, 40% or more, 50% or more, 60% or more, 70% or more, 80% or more 90% or more) as compared to the level of IL-1ra determined in step (e) demonstrates that the subject will benefit from said therapeutic treatment.

In the described method, a reduction of the level of MCP-1 determined in step (d) by 10% or more (e.g. 20% or more, 30% or more, 40% or more, 50% or more, 60% or more, 70% or more, 80% or more 90% or more) as compared to the level of MCP-1 determined in step (e) demonstrates that the subject will benefit from said therapeutic treatment.

In the described method, a reduction of the level of VEGF determined in step (d) by 10% or more (e.g. 20% or more, 30% or more, 40% or more, 50% or more, 60% or more, 70% or more, 80% or more 90% or more) as compared to the level of VEGF determined in step (e) demonstrates that the subject will benefit from said therapeutic treatment.

In the described method, a reduction of the level of VCAM-1 determined in step (d) by 10% or more (e.g. 20% or more, 30% or more, 40% or more, 50% or more, 60% or more, 70% or more, 80% or more 90% or more) as compared to the level of VCAM-1 determined in step (e) demonstrates that the subject will benefit from said therapeutic treatment.

In the described method, the cancer is selected from the group consisting of primary pancreatic cancer, metastatic pancreatic cancer, primary colorectal cancer, metastatic colorectal cancer, primary ovarial cancer, metastatic ovarial cancer, primary renal cell cancer, metastatic renal cell cancer, primary stomach cancer, metastatic stomach cancer, primary prostate cancer, metastatic prostate cancer, primary breast cancer, metastatic breast cancer, primary hepatocellular cancer, metastatic hepatocellular cancer, primary lung cancer, metastatic lung cancer, primary head and neck cancer, metastatic head and neck cancer, primary gastric cancer, metastatic gastric cancer, primary esophageal cancer, and metastatic esophageal cancer.

In the described method, the cancer can be in very early stages, from pre-malignant lesions, through primary tumors (non-metastatic) to metastatic stages (primary tumors with lymph node involvement, vessel invasion, lymph vessel invasion, distant organ metastases of all sites). In one embodiment of the fourth aspect, the cancer only appears as metastasis without a designated primary tumor (cancer of unknown primary). The cancer to be treated according to the present invention is metastatic colorectal cancer.

In the described method, the CCR5 antagonist is selected from the group consisting of Maraviroc (Pfizer), Vicriviroc (Schering-Plough), Aplaviroc (GlaxoSmithKline(GSK)), ancriviroc, [3H]maraviroc, [3H]ancriviroc, CCL7, TAK-779, E913, TAK-652, TAK-220, and vMIP-II. Only maraviroc and [3H]maraviroc are CCR5 antagonists of the present invention.

The following examples and drawings illustrate the present invention without, however, limiting the same thereto.

### EXAMPLES

The following examples are put forth so as to provide those of ordinary skill in the art with a complete disclosure and description of how to make and use the methods and compositions of the invention, and are not intended to limit the scope of what the inventors regard as their invention. Efforts have been made to ensure accuracy with respect to numbers used but some experimental errors and deviations should be accounted for.

### Materials and Methods

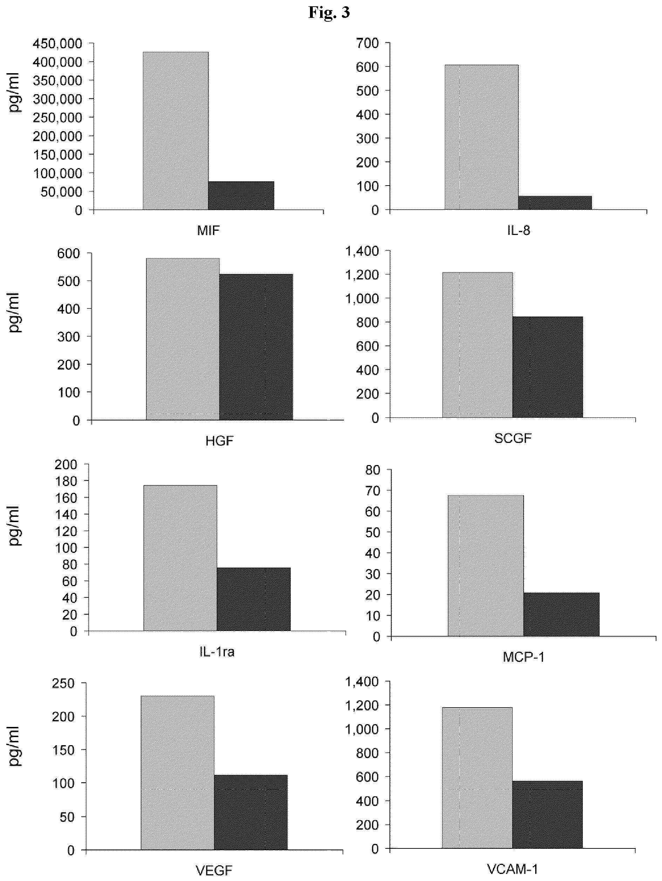

**Tumor explant models.** Tumor explant models were generated from freshly resected colorectal cancer liver metastases. Explanted tissue specimens were cut into three equal parts, placed in unconditioned medium and either treated with DMSO or Maraviroc (in DMSO) or without any addition. The explants were then harvested and subjected to histological analyses (see Figure 1) and multiplex protein analysis (see Figure 3).

**Clinical trial.** A trial in colorectal cancer patients with hepatic metastases was initiated. Patients had to have had all standard of care treatment for colorectal cancer (mainly FOLFOX, FOLFIRI, Cetuximab, Bevacizumab, 5-FU etc.). The MARACON-001 phase I trial (*"Treatment of Advanced Colorectal Cancer Patients with Hepatic Liver Metastases using the CCRS-Antagonist Maraviroc"*) was registered at clinicaltrials.gov (identifier NCT01736813) and at the European clinical trial registry (EudraCT 2012-000861-18). In the trial, patients receive Maraviroc daily for two months. Safety and feasibility are the primary endpoints of this ongoing trial. The trial is being conducted according to the Declaration of Helsinki and relevant International Conference on Harmonization Good Clinical Practice guidelines and with approval from the ethics committee of the University of Heidelberg. All patients had received all current standard of care treatment options and were now refractory to standard chemotherapy. All provided written informed consent before participating in this study. The overall workflow of the trial is depicted in Figure 2.

**Assessment of proliferation in histology samples.** Briefly cryo sections were fixed with 4 % paraformaldehyde (PFA, Sigma Aldrich, Germany). The sections were blocked with 10% normal goat serum (Vector, USA). Ki67 primary antibody (clone MIB-1, DAKO, USA, 1:200) was applied at room temperature for 30 minutes. The slides were incubated with a secondary antibody (rabbit-anti-mouse IgG, Bond Polymer Refine Detection System, Leica, Germany) for 8 minutes at room temperature. Further amplification of the signal was achieved through incubation with a third antibody, conjugated with horse radish peroxidase and coupled to dextrane molecules in large numbers, for 8 minutes at room temperature (Poly-HRP-mouse-anti-rabbit IgG, Bond Polymer Refine Detection System, Leica, Germany). The antigen detection was performed by a color reaction with 3,3-di-amino-benzidine (DAB chromogen, Bond Polymer Refine Detection System, Leica, Germany). The sections were counterstained with hematoxylin (Bond Polymer Refine Detection System, Leica, Germany) and mounted with Aquatex (Merck, Germany). Assessment was performed with the Visiomorph Software Package (Visiopharm, Denmark) to quantify positive tumor cells.

**CCR5 staining of tumor tissue.** Tissue blocks were cut and tissue sections (4 µm) were prepared from formalin-fixed, paraffin-embedded tissue. Following deparaffinization and rehydration, slides were transferred to a fully automated staining facility (Leica BOND-MAX^{™}, Leica Microsystems, Germany). After heat-induced epitope retrieval (HIER1) at 100°C (BOND Epitope Retrieval Solution 1, Leica, Germany) the endogenous peroxidase activity was blocked by incubation with 0.6% H₂O₂ in methanol for 10 minutes. The sections were blocked with 10% normal goat serum (Vectastain^{®} Elite ABC kit, Vector, USA). A mouse monoclonal antibody recognizing human CCR5 (1:50 dilution, clone MM0065-6H20, Abcam, USA) was applied as primary antibody at room temperature for 30 minutes. The slides were incubated with a secondary antibody (rabbit-anti-mouse IgG, Bond Refine Detection Kit, Leica, Germany) for 8 minutes at room temperature. Further amplification of the signal was achieved through incubation with a third antibody, conjugated with horse radish peroxidase and coupled to dextrane molecules in large numbers, for 8 minutes at room temperature (mouse-anti-rabbit IgG, Bond Refine Detection Kit, Leica, Germany). The antigen detection was performed by a color reaction with 3,3-di-amino-benzidine (DAB+ chromogen, Leica, Germany). The sections were counterstained with hematoxylin (Leica, Germany) and mounted with Aquatex (Merck, Germany).

### Results

**Inhibition of CCR5 with Maraviroc leads to tumor cell death in explant models and in patients.** Data from multiplex explant models showed clear tumor cell death upon inhibition of CCR5 while no effects were seen in the adjacent liver (see Figure 1). The MARACON trial was initiated and results from the biopsies confirmed these findings.

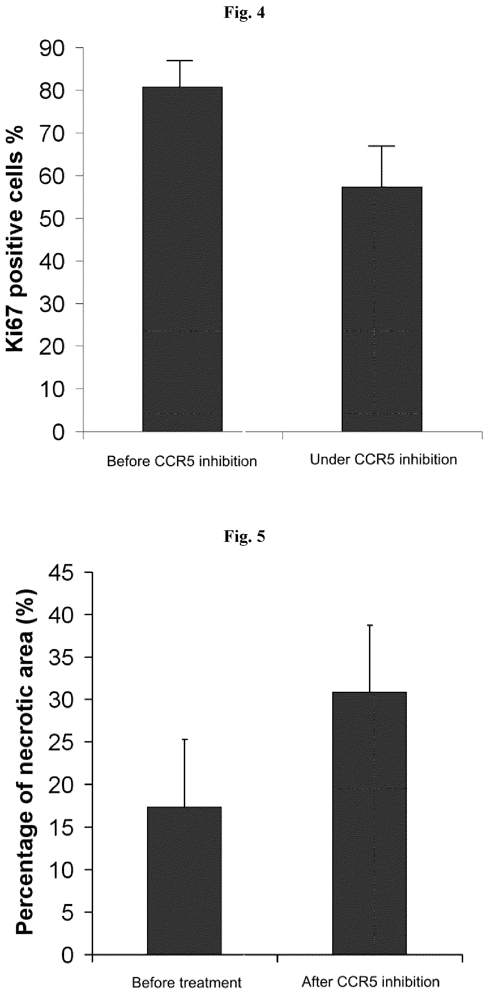

**Complex changes in the microenvironment ameliorate the immunosuppressive microenvironment and lead to reduced tumor cell growth.** The resulting changes in the cytokine composition in the biopsy material showed dramatic improvements of the immunological microenvironment. Also angiogenesis and other growth factors were reduced (see Figure 3). This was accompanied by reduced proliferation of tumor cells and tumor cell death (see Figure 4).

**Objective responses in patients from the trial and in patients from other cancer entities show favorable clinical effects.** Patients from the MARACON trial (monotherapy) as well as patients receiving combination therapy showed objective responses to CCR5 inhibition. Across varying tumor entities and after different multiple previous therapies, patients could achieve responses with CCR5 inhibition (see Table 1). Furthermore, signs of cytostatic effects (i.e. halted or slowed tumor growth as witnessed by increased necrosis within the metastasis) were visible on radiologic image analyses (see Figure 5).

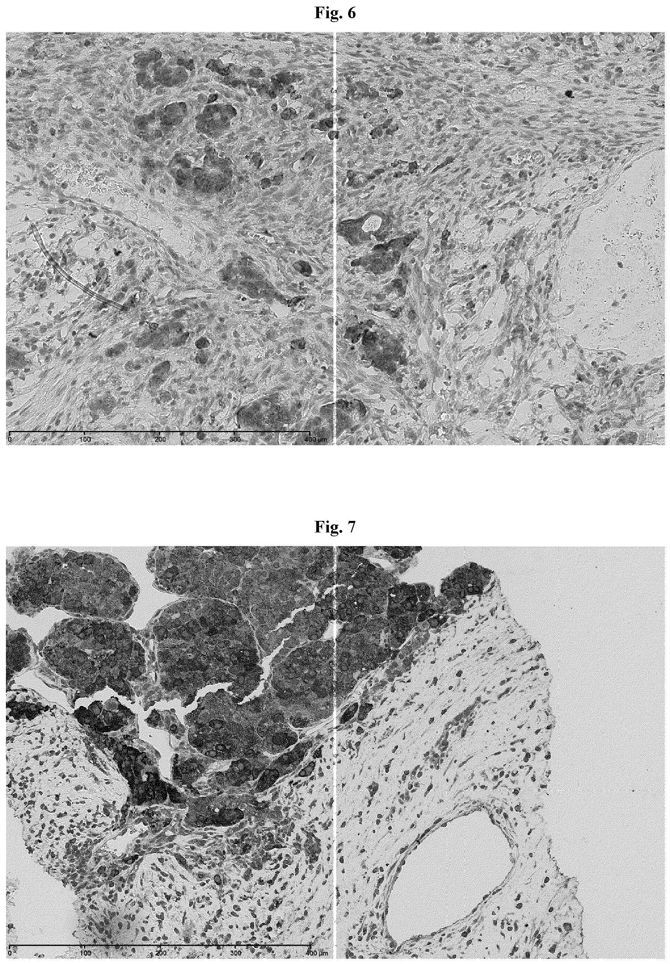

**CCR5 is also expressed on other tumor entities.** Immunohistochemical analyses revealed that CCR5 is also expressed on other tumor types. Data was obtained for prostate (data not shown), gastric (Figure 6) and hepatocellular cancer (Figure 7). Therefore it seems most likely that these tumor entities allow for effective blockade of CCR5 as in colorectal cancer.

**Table 1. Clinical responses in patients treated with Maraviroc (as monotherapy or in combination therapy)**

| **Patient No.** | **Cancer entity** | **Monotherapy** | **Combination therapy** | **Tumor marker** | **Objective response (RECIST) / other responses** |
|---|---|---|---|---|---|
| 1 | Pancreatic cancer (metastatic: liver, lung, bone, peritoneal) | No | Gemcitabine, Paclitaxel (albumin bound) and Maraviroc | Reduced to 10% | -35% |
| 2 | colorectal cancer (metastatic: liver) | No | FOLFOX4, Bevacizumab and Maraviroc | Reduced to 12% | -76% |
| 3 | colorectal cancer (metastatic: liver and peritoneal) | No | FOLFOX4, Bevacizumab and Maraviroc | Reduced to 21% | -48% |
| 4 | Colorectal cancer (metastatic: lung and liver) | Yes | No | Not available | -50% (lung), -10% (liver) |
| 5 | Breast cancer (metastatic: liver) | No | Paclitaxel, Bevacizumab and Maraviroc | Unchanged | -30% |
| 6 | Ovarial cancer (metastatic: liver, lungs, abdomen) | No | Carboplatin (dose-reduced) and Maraviroc | Reduced to 50% | -25% |

### INDUSTRIAL APPLICABILITY OF THE PRESENT INVENTION

The present application shows our finding that the blockade of CCR5 in humans leads to significant anti-tumor effects and an altered microenvironment in colorectal cancer liver metastases. Tumor cells have CCR5, the receptor for CCL5, which upon binding leads to increased proliferation and invasive properties of the tumor cells and therefore exploit the host's immune system.

We confirmed our findings in a phase I clinical trial using the CCR5 inhibitor Maraviroc in a cohort of chemotherapy refractory colorectal cancer patients and could verify our hypothesis based on sequential biopsies of treated patients *in vivo,* showing clinical responses as well as drastic immunological effects on the tissue level.

Further on we noted that combination therapy of a CCR5 inhibitor with other treatments (chemotherapy, radiation etc.) can induce responses in these patients, even if combinations include treatment regimens that were previously ineffective. The addition of CCR5 blockade to these treatments enhances efficacy and leads to objective responses.

We then applied this approach for other cancer entities and observed similar effects for combination therapies of CCR5 inhibition and other treatments. Effects were seen in pancreatic cancer, colorectal cancer, breast cancer and ovarial cancer.

As the CCR5 inhibitor Maraviroc is well-tolerated and does not have significant side effects so far, the outlook for monotherapies and combination therapies (i.e. an addition of chemotherapy, vaccination, immunotherapy or immunostimulating agents) is very promising, wherein the early data from patients shown herein strongly support this observation.

### REFERENCES

Berger EA, Murphy PM, Farber JM. Chemokine receptors as HIV-1 coreceptors: roles in viral entry, tropism, and disease. Annu Rev Immunol. 1999; 17:657-700.
Dorr P, Westby M, Dobbs S, Griffin P, Irvine B, Macartney M, Mori J, Rickett G et al. Maraviroc (UK-427,857), a potent, orally bioavailable, and selective small-molecule inhibitor of chemokine receptor CCR5 with broad-spectrum anti-human immunodeficiency virus type 1 activity. Antimicrob Agents Chemother. 2005; 49(11):4721-32.
Haworth B, Lin H, Fidock M, Dorr P, Strange PG. Allosteric effects of antagonists on signalling by the chemokine receptor CCR5. Biochem Pharmacol. 2007; 74(6):891-7.
Musha H, Ohtani H, Mizoi T, Kinouchi M, Nakayama T, Shiiba K, Miyagawa K, Nagura H et al. Selective infiltration of CCR5(+)CXCR3(+) T lymphocytes in human colorectal carcinoma. Int J Cancer. 2005; 116(6):949-56.
Ohtani H. Focus on TILs: prognostic significance of tumor infiltrating lymphocytes in human colorectal cancer. Cancer Immun. 2007; 7:4.
Oppermann M. Chemokine receptor CCR5: insights into structure, function, and regulation. Cell Signal. 2004; 16(11):1201-10.
Wong MM, Fish EN. Chemokines: attractive mediators of the immune response. Semin Immunol. 2003; 15(1):5-14.

## Claims

1. A CCR5 antagonist for use in the treatment of metastatic colorectal cancer in a subject receiving immunomodulatory therapy with anti-PD-1,
wherein the CCR5 antagonist is Maraviroc or [3H]maraviroc.

2. The CCR5 antagonist for use according to claim 1, wherein said subject receives at least one further anti-cancer-therapy selected from the group consisting of
- chemotherapy,
- radiation therapy,
- vaccination,
- immunotherapy,
- stem cell therapy,
- anti-hormonal therapy,
- local ablation of metastases or tumors by physical intervention, and
- combinations of any of the above.

3. The CCR5 antagonist for use according to claim 2,
wherein the chemotherapy comprises the administration of an alkylating agent, an antimetabolite, folinic acid, a folate antagonist, a mitotic inhibitor, an anthracyclin, a topoisomerase inhibitor, an antibody, a signal transduction inhibitor, an inhibitor of angiogenesis, and an inhibitor of histone deacetylase,
wherein the alkylating agent preferably is selected from the group consisting of bendamustin, busulfan, carboplatin, carmustin, cisplatin, oxaliplatin, cyclophosphamid, mitomycin, and treosulfan,
wherein the antimetabolite preferably is selected from the group consisting of 5-fluorouracil, capecitabin, cytarabin, gemcitabin, mercaptopurin, and deoxyglucose,
wherein the folate antagonist preferably is selected from the group consisting of methotrexate and pemetrexed,
wherein the mitotic inhibitor preferably is selected from the group consisting of taxanes and vinca-alcaloids,
wherein the taxane preferably is selected from the group consisting of cabzitaxel, docetaxel, and paclitaxel,
wherein the anthracyclin preferably is selected from the group consisting of bleomycin, doxorubicin, mitoxantron, and epirubicin,
wherein the topoisomerase inhibitor preferably is selected from the group consisting of campthotecin-derivatives and podophyllin-derivatives,
wherein the campthotecin-derivative preferably is selected from the group consisting of irinotecan and topotecan,
wherein the antibody preferably is selected from the group consisting of bevacizumab (Avastin), cetuximab (Erbitux), panitumumab (Vectibix), ipilimumab, nivulomumab, tremelimumab, anti-OX40-antibodies, catumaxomab, and anti-CD40L antibodies,
wherein the signal transduction inhibitor preferably is selected from the group consisting of axatinib, crizotinib, erlotinib, sunitinib, sorafenib, everolimus, imatinib, lapatinib, pazopanib, temsirolimus, and vemurafenib,
wherein the inhibitor of angiogenesis preferably is aflibercept, or
wherein the inhibitor of histone deacetylase preferably is vorinostat.

4. The CCR5 antagonist for use according to any one of claims 1 to 3, wherein said CCR5 antagonist is to be administered in an amount of between about 150 mg and about 600 mg once or twice a day.

5. The CCR5 antagonist for use according to any one of claims 1 to 4, wherein said use is as a tumor cell death-inducing medicament.

6. A CCR5 antagonist for use in the treatment of metastases of colorectal carcinoma by inducing tumor cell death, wherein said CCR5 antagonist is Maraviroc or [3H]maraviroc.

7. The CCR5 antagonist for use according to claim 6, wherein the metastases are selected from the group consisting of liver metastases, lung metastases, bone metastases, pancreas metastases, lymph node metastases, and peritoneal metastases.

8. The CCR5 antagonist for use according to claim 6 or 7, wherein said CCR5 antagonist is to be administered in an amount of between about 150 mg and about 600 mg once or twice a day.

9. The CCR5 antagonist for use according to claim 2, wherein the CCR5 antagonist is combined with
(i) radiation therapy; or
(ii) chemotherapy and radiation therapy.

10. The CCR5 antagonist for use according to claim 9 (i), wherein the CCR5 antagonist is combined with radiation therapy consisting of low dose radiation with 2-30 Gy doses or high dose radiation with 31-100 Gy radiation.

11. The CCR5 antagonist for use according to claim 9 (ii), wherein the CCR5 antagonist is combined with chemotherapy consisting of platinum-based therapies (e.g. FOLFOX), gemcitabine, or taxanes (e.g. paclitaxel), and radiation therapy consisting of low dose radiation with 2-30 Gy doses or high dose radiation with 31-100 Gy radiation.

12. The CCR5 antagonist for use according to any one of claims 9-11, wherein the treatment modalities are used sequentially or concurrent.

## Patentansprüche

1. CCR5-Antagonist zur Verwendung bei der Behandlung von metastasierendem Kolorektalkrebs bei einem Subjekt, das eine immunmodulatorische Therapie mit Anti-PD-1 erhält,
wobei der CCR5-Antagonist Maraviroc oder [3H]Maraviroc ist.

2. CCR5-Antagonist zur Verwendung nach Anspruch 1, wobei das Subjekt mindestens eine weitere Antikrebstherapie erhält, die aus der folgenden Gruppe ausgewählt ist:
- Chemotherapie,
- Strahlentherapie,
- Impfung,
- Immuntherapie,
- Stammzelltherapie,
- antihormonelle Therapie,
- lokale Ablation von Metastasen oder Tumoren durch physische Intervention und
- Kombinationen aus beliebigen der oben genannten.

3. CCR5-Antagonist zur Verwendung nach Anspruch 2, wobei die Chemotherapie die Verabreichung von einem Alkylierungsmittel, einem Antimetaboliten, Folinsäure, einem Folatantagonisten, einem Mitosehemmer, einem Anthracyclin, einem Topoisomerasehemmer, einem Antikörper, einem Signaltransduktionsinhibitor, einem Angiogenesehemmer und einem Histondeacetylasehemmer umfasst,
wobei das Alkylierungsmittel vorzugsweise aus der aus Bendamustin, Busulfan, Carboplatin, Carmustin, Cisplatin, Oxaliplatin, Cyclophosphamid, Mitomycin und Treosulfan bestehenden Gruppe ausgewählt ist,
wobei der Antimetabolit vorzugsweise aus der aus 5-Fluoruracil, Capecitabin, Cytarabin, Gemcitabin, Mercaptopurin und Desoxyglucose bestehenden Gruppe ausgewählt ist,
wobei der Folatantagonist vorzugsweise aus der aus Methotrexat und Pemetrexed bestehenden Gruppe ausgewählt ist,
wobei der Mitosehemmer vorzugsweise aus der aus Taxanen und Vinca-Alkaloiden bestehenden Gruppe ausgewählt ist,
wobei das Taxan vorzugsweise aus der aus Cabzitaxel, Docetaxel und Paclitaxel bestehenden Gruppe ausgewählt ist,
wobei das Anthracyclin vorzugsweise aus der aus Bleomycin, Doxorubicin, Mitoxantron und Epirubicin bestehenden Gruppe ausgewählt ist,
wobei der Topoisomerasehemmer vorzugsweise aus der aus Campthotecinderivaten und Podophyllinderivaten bestehenden Gruppe ausgewählt ist,
wobei das Campthotecinderivat vorzugsweise aus der aus Irinotecan und Topotecan bestehenden Gruppe ausgewählt ist,
wobei der Antikörper vorzugsweise aus der aus Bevacizumab (Avastin), Cetuximab (Erbitux), Panitumumab (Vectibix), Ipilimumab, Nivulomumab, Tremelimumab, Anti-OX40-Antikörpern, Catumaxomab und Anti-CD40L-Antikörpern bestehenden Gruppe ausgewählt ist,
wobei der Signaltransduktionsinhibitor vorzugsweise aus der aus Axatinib, Crizotinib, Erlotinib, Sunitinib, Sorafenib, Everolimus, Imatinib, Lapatinib, Pazopanib, Temsirolimus, und Vemurafenib bestehenden Gruppe ausgewählt ist,
wobei der Angiogenesehemmer vorzugsweise Aflibercept ist oder
wobei der Histondeacetylasehemmer vorzugsweise Vorinostat ist.

4. CCR5-Antagonist zur Verwendung nach einem der Ansprüche 1 bis 3, wobei der CCR5-Antagonist einmal oder zweimal täglich in einer Menge zwischen etwa 150 mg und etwa 600 mg zu verabreichen ist.

5. CCR5-Antagonist zur Verwendung nach einem der Ansprüche 1 bis 4, wobei die Verwendung als ein tumorzelltodinduzierendes Medikament dient.

6. CCR5-Antagonist zur Verwendung bei der Behandlung von Metastasen eines Kolorektalkarzinoms durch Induzieren des Tumorzelltodes, wobei der CCR5-Antagonist Maraviroc oder [3H]Maraviroc ist.

7. CCR5-Antagonist zur Verwendung nach Anspruch 6, wobei die Metastasen aus der aus Lebermetastasen, Lungenmetastasen, Knochenmetastasen, Pankreasmetastasen, Lymphknotenmetastasen und Peritonealmetastasen bestehenden Gruppe ausgewählt sind.

8. CCR5-Antagonist zur Verwendung nach Anspruch 6 bis 7, wobei der CCR5-Antagonist einmal oder zweimal täglich in einer Menge zwischen etwa 150 mg und etwa 600 mg zu verabreichen ist.

9. CCR5-Antagonist zur Verwendung nach Anspruch 2, wobei der CCR5-Antagonist kombiniert wird mit
(i) Strahlentherapie oder
(ii) Chemotherapie und Strahlentherapie.

10. CCR5-Antagonist zur Verwendung nach Anspruch 9 (i), wobei der CCR5-Antagonist mit einer Strahlentherapie kombiniert wird, die aus einer niedrigdosierten Strahlung mit 2-30-Gy-Dosen oder einer hochdosierten Strahlung mit 31-100-Gy-Strahlung besteht.

11. CCR5-Antagonist zur Verwendung nach Anspruch 9 (ii), wobei der CCR5-Antagonist mit Chemotherapie, bestehend aus platinbasierten Therapien (z. B. FOLFOX), Gemcitabin oder Taxanen (z. B. Paclitaxel), und Strahlentherapie, bestehend aus niedrigdosierter Strahlung mit 2-30-Gy-Dosen oder hochdosierter Strahlung mit 31-100-Gy-Strahlung, kombiniert wird.

12. CCR5-Antagonist zur Verwendung nach einem der Ansprüche 9-11, wobei die Behandlungsmodalitäten aufeinanderfolgend oder gleichzeitig angewendet werden.

## Revendications

1. Antagoniste de CCR5 pour utilisation dans le traitement du cancer colorectal métastatique chez un sujet recevant une thérapie immunomodulatrice par anti-PD-1,
l'antagoniste de CCR5 étant le maraviroc ou le [³H]maraviroc.

2. Antagoniste de CCR5 selon la revendication 1, ledit sujet recevant au moins une autre thérapie anticancéreuse choisie dans le groupe constitué par
- une chimiothérapie,
- une radiothérapie,
- une vaccination,
- une immunothérapie,
- une thérapie par cellules souches,
- une thérapie antihormonale,
- une ablation locale de métastases ou de tumeurs par intervention physique, et
- des combinaisons quelconques de celles-ci.

3. Antagoniste de CCR5 selon la revendication 2,
la chimiothérapie comprenant l'administration d'un agent alkylant, d'un antimétabolite, d'acide folinique, d'un antagoniste des folates, d'un inhibiteur mitotique, d'une anthracycline, d'un inhibiteur de la topoisomérase, d'un anticorps, d'un inhibiteur de transduction de signal, d'un inhibiteur de l'angiogenèse et d'un inhibiteur de l'histone désacétylase,
l'agent alkylant étant de préférence choisi dans le groupe constitué par la bendamustine, le busulfan, le carboplatine, la carmustine, le cisplatine, l'oxaliplatine, le cyclophosphamide, la mitomycine et le tréosulfan,
l'antimétabolite étant de préférence choisi dans le groupe constitué par le 5-fluorouracile, la capécitabine, la cytarabine, la gemcitabine, la mercaptopurine, et le désoxyglucose, l'antagoniste des folates étant de préférence choisi dans le groupe constitué par le méthotrexate et le pémétrexed,
l'inhibiteur mitotique étant de préférence choisi dans le groupe constitué par les taxanes et les alcaloïdes de vinca,
le taxane étant de préférence choisi dans le groupe constitué par le cabzitaxel, le docétaxel et le paclitaxel,
l'anthracycline étant de préférence choisie dans le groupe constitué par la bléomycine, la doxorubicine, le mitoxantron et l'épirubicine,
l'inhibiteur de topoisomérase étant de préférence choisi dans le groupe constitué par les dérivés de campthotécine et les dérivés de podophylline,
le dérivé de campthotécine étant de préférence choisi dans le groupe constitué par l'irinotécan et le topotécan,
l'anticorps étant de préférence choisi dans le groupe constitué par le bévacizumab (Avastin), le cétuximab (Erbitux), le panitumumab (Vectibix), l'ipilimumab, le nivulomumab, le trémélimumab, des anticorps anti-OX40, le catumaxomab et des anticorps anti-CD40L,
l'inhibiteur de transduction de signal étant de préférence choisi dans le groupe constitué par l'axatinib, le crizotinib, l'erlotinib, le sunitinib, le sorafénib, l'évérolimus, l'imatinib, le lapatinib, le pazopanib, le temsirolimus et le vémurafénib,
l'inhibiteur de l'angiogenèse étant de préférence l'aflibercept, ou
l'inhibiteur de l'histone désacétylase étant de préférence le vorinostat.

4. Antagoniste de CCR5 selon l'une quelconque des revendications 1 à 3, ledit antagoniste de CCR5 devant être administré en une quantité comprise entre environ 150 mg et environ 600 mg une ou deux fois par jour.

5. Antagoniste de CCR5 selon l'une quelconque des revendications 1 à 4, ladite utilisation étant en tant qu'un médicament induisant la mort des cellules tumorales.

6. Antagoniste de CCR5 pour une utilisation dans le traitement des métastases de carcinome colorectal par induction de la mort des cellules tumorales, ledit antagoniste de CCR5 étant le maraviroc ou le [3H]maraviroc.

7. Antagoniste de CCR5 selon la revendication 6, les métastases étant choisies dans le groupe constitué par les métastases hépatiques, les métastases pulmonaires, les métastases osseuses, les métastases pancréatiques, les métastases ganglionnaires et les métastases péritonéales.

8. Antagoniste de CCR5 selon la revendication 6 ou 7, ledit antagoniste de CCR5 devant être administré en une quantité comprise entre environ 150 mg et environ 600 mg une ou deux fois par jour.

9. Antagoniste de CCR5 selon la revendication 2, l'antagoniste de CCR5 étant associé à
(i) une radiothérapie ; ou
(ii) une chimiothérapie et une radiothérapie.

10. Antagoniste de CCR5 selon la revendication 9 (i), l'antagoniste de CCR5 étant associé à une radiothérapie comprenant un rayonnement à faible dose avec des doses de 2 à 30 Gy ou un rayonnement à dose élevée avec un rayonnement de 31 à 100 Gy.

11. Antagoniste de CCR5 selon la revendication 9 (ii), l'antagoniste de CCR5 étant associé à une chimiothérapie comprenant des traitements à base de platine (par exemple, FOLFOX), la gemcitabine ou les taxanes (par exemple, le paclitaxel), et une radiothérapie comprenant un rayonnement à faible dose avec des doses de 2 à 30 Gy ou un rayonnement à dose élevée avec un rayonnement de 31 à 100 Gy.

12. Antagoniste de CCR5 selon l'une quelconque des revendications 9 à 11, les modalités de traitement étant utilisées de façon séquentielle ou concomitante.
